# EUROPEAN PATENT APPLICATION

(11) **EP 0 635 545 A2**
(43) Date of publication of application: **25.01.1995**
(21) Application number: 94111240.1
(22) Date of filing: 19.07.1994
(51) Int. Cl.: C08L 29/04, C08K 5/053, A61F 13/20

(54) **Injection molded articles from extrudable polyvinyl alcohol compositions**

(30) Priority: 21.07.1993 US 95635
(71) Applicant: AIR PRODUCTS AND CHEMICALS, INC., Allentown, PA 18195-1501 (US)
(72) Inventor: Famili, Amir, Orefield, PA 18069 (US); Marten, Finn Lennart, Emmaus, PA 18049 (US); Nangeroni, James Francis, Sparta, NJ 07871 (US)
(74) Representative: Kador & Partner

(57) **Abstract**

An article of manufacture comprising a thermoformed polyvinyl alcohol compounded with a solid plasticizer and a liquid plasticizer, the polyvinyl alcohol made melt extrudable by adding sufficient energy to the polyvinyl alcohol to both melt it and substantially eliminate the crystallinity in the melt while simultaneously removing energy from the melt at a rate sufficient avoid decomposition of the polyvinyl alcohol and the solid plasticizer being sorbitol, mannitol, pentaerythritol and/or dipentaerythritol.

## Description

### FIELD OF THE INVENTION

The present invention relates to melt extrudable polyvinyl alcohol compositions useful for injection molding of articles, particularly personal care articles which are flushable and biodegradable.

### BACKGROUND OF THE INVENTION

Polyvinyl alcohol (PVOH) is a water soluble, repulpable and biodegradable resin with excellent aroma and oxygen barrier properties and resistance to most organic solvents. The polymer is used extensively in adhesive, textile sizing and paper coating. However, the end uses of PVOH have been limited despite its excellent mechanical, physical and chemical properties to those uses in which it is supplied as a solution in water. This limitation is partly due to the fact that the vinyl alcohol polymers in the unplasticized state have a high degree of crystallinity and show little or no thermoplasticity before the occurrence of decomposition which starts at about 107°C and becomes pronounced at 200°C, which is well below its crystalline melting point.

U.S. 5,051,222 discloses a method for making thermoplastic (melt extrudable) PVOH compositions which comprises providing sufficient energy to a PVOH which is at least partially crystalline to both melt the PVOH and substantially eliminate the crystallinity in the PVOH melt while simultaneously removing energy from the PVOH melt at a rate sufficient to avoid decomposition of the PVOH. The melt is then extruded, preferably into a strand, rapidly cooled and cut into pellets for subsequent thermoprocessing into the desired product or article.

U.S. 4,618,648 discloses a modified self-plasticizing poly(vinyl alcohol) which is a poly(vinyl alcohol)-co-poly(alkyleneoxy) acrylate.

In the present public concern for preserving the environment, there is a search for materials which, when disposed of after use, will physically or biologically decompose and thereby avoid polluting the environment. One logical candidate for such a material is water soluble PVOH. The desirable physical and mechanical properties of PVOH such as biodegradability, water solubility and repulpability combined with thermoplasticity have opened a new market opportunity for PVOH. An example includes packaging applications in which PVOH film is used for packaging pesticides, caustic cleaners and detergents. These packages are dissolved in water during use thereby eliminating human exposure to high toxic or concentrated chemicals and the need to clean and discard a contaminated chemical container after use. The PVOH films are also used to control contamination in hospitals. Soiled laundry is collected and placed directly into the bag. The bag and the contents are then placed directly into the washing machine where the bag will dissolve completely.

There are several U.S. patents disclosing injecting molding of thermoplastic PVOH for disposable sanitary products such as tampon applicators. Tampon applicators in current commercial use are made either from paper or plastic. The plastic applicators are normally preferred by many women because of comfort. These applicators are typically disposed of in the toilet bowl where it either settles in the septic tank without decomposing or finds its way to sewer treatment plants. If the treatment plant fails to remove these applicators, they could end up in the environment, especially on beaches. Therefore, it is highly desirable to produce a flushable, water dispersible and biodegradable tampon applicator.

The thermoplastic PVOH resins of U.S. 5,051,222 give molded parts that are stiff when removed from the molding machine but pick up moisture from the atmosphere and become too flexible for machine handling in the manufacture of tampon applicators.

U.S. 4,469,837 discloses a process for producing film from PVOH and pentaerythritol by non-aqueous hot melt extrusion.

EP 0 236 011 discloses a water-soluble, moistureproof film of PVOH comprising a PVOH and 5 to 20 wt%, based on PVOH, of pentaerythritol, the film having been produced by the casting method or the melt-extrusion method using water.

U.S. 3,882,869 discloses a water-dispersible insertion device for tampons comprising tubes made from thermoplastic water soluble polymer compounded with water-insoluble fillers.

U.S. 3,911,917 discloses a disposable injector device for inserting tampons. The device is made from heat-degraded thermoformed water-soluble PVOH which is rendered odor-free by mixing a proton acceptor with the PVOH before the PVOH is heat degraded by a thermoforming operation.

U.S. 4,372,311 discloses disposable articles made from a water soluble polymer or a substrate made from such polymer, a surface of said article or substrate coated with a degradable water-insoluble polymer.

U.S. 5,002,526 discloses a tampon applicator which is biodegradable and water-dispersible which is formed by injection molding a modified poly(vinyl alcohol) that is self-plasticizing.

### SUMMARY OF THE INVENTION

The present invention provides a thermoformable PVOH composition which can be melt extruded or injection molded into a thin wall structure which is flushable and biodegradable while retaining its wall integrity and stiffness at high humidity. The thermoformable PVOH composition consists essentially of a thermoplastic PVOH, a solid plasticizer for PVOH, a liquid plasticizer for PVOH and, optionally, a filler to reduce water sensitivity and increase the modulus of the product. The solid plasticizer at the temperatures in the melt stage acts as a plasticizer for PVOH while at room temperature (20-25°C) acts as a filler to raise the modulus of the polymer composition.

More specifically, the composition consists essentially of (a) thermoplastic PVOH, (b) 1-10 wt% of a solid plasticizer, based on PVOH, (c) 8-20 wt% liquid plasticizer, based on PVOH, and (d) 0-50 wt% of a filler, based on PVOH.

This composition is readily melt extrudable and provides an ability to thermoform (especially by injection molding) articles, preferably thin wall articles such as tampon applicators. Such thin wall articles demonstrate reduced moisture sensitivity, improved modulus and reduced elongation at high relative humidity. By "thin wall" we mean a structure having a wall 1/30 to 1/16 inches (0.85 to 1.59 mm) in thickness.

Also provided is a process for the production of such thermoprocessable compositions in the form of essentially gel-free thermoplastic pellets useful for conventional thermoplastic processing including injection molding and melt extrusion.

The pellets or the thermoformed article can be obtained by providing sufficient energy to the melt extrudable PVOH composition to both melt the PVOH and substantially eliminate the crystallinity in the PVOH melt while simultaneously removing energy from the PVOH melt at a rate sufficient to avoid decomposition of the PVOH melt. The melt is then extruded into the desired article or into a strand, rapidly cooled and cut into pellets for subsequent thermoprocessing to the desired article.

The energy input utilized in the process is at least about 0.27 KW hr/kg PVOH and desirably less than 0.6 KW hr/kg PVOH, preferably 0.3 to 0.45 KW hr/kg PVOH. Additional energy would be required to melt extrude or injection mold the pellets into the desired article.

According to the present invention, the necessary energy is added to melt the PVOH and additional energy is added to sheer the areas of PVOH crystallinity in the melt, while at the same time removing the sheering energy to prevent the melt temperature from exceeding the PVOH decomposition of temperature. That is to say, the additional energy added to effect the sheering essentially passes through the resin melt eliminating the crystallinity and is removed by simultaneously cooling the melt, for example via the cooling jacket of the extruder.

The extruder requires intensive mixing elements to provide the requisite sheering energy. The sheering energy generated in a particular zone of the extruder should not be greater than that which can be removed by cooling; otherwise decomposition results.

### DETAILED DESCRIPTION OF THE INVENTION

Suitable PVOHs for use in the preparation of the extrudable PVOH composition include those PVOH's which are at least partially crystalline and are at least 75 mole% hydrolyzed, preferably 85-99 mole% hydrolyzed, and possess a degree of polymerization (DPn) in the range of 200-2500, i.e., solution viscosities of 3-55 cps at 20°C as a 4% aqueous solution, preferably a DPn from 200 to 1000.

For making thin wall injection molded articles such as tampon applicators, a composition containing only fully hydrolyzed PVOH would be very brittle and the use of more plasticizer to offset the brittleness would lead to lower water resistance. On the other hand, an all partially hydrolyzed PVOH composition would be too tacky in use and too soft for the manufacturing process. Thus, a blend of partially and fully hydrolyzed PVOH's is preferably used. Such blend comprises 30-50 wt% partially hydrolyzed (85-90 mole%) PVOH and 50-70 wt% fully hydrolyzed (at least 98 mole%) PVOH, especially a 40/60 wt blend of partially/fully hydrolyzed PVOH's.

The PVOH may also contain up to 3-5 mole% of a copolymerized monomer. For example, copolymers of vinyl alcohol and methyl methacrylate consisting of 94-98 mole% vinyl alcohol and 2-6 wt% methyl methacrylate as disclosed in U.S. 3,689,469 are considered suitable equivalents to the vinyl alcohol/vinyl acetate copolymers containing the same mole% acetate units.

The thermoformable PVOH composition should have a water content of less than about 4 wt%, preferably less than about 2 wt% as is present in commercially available PVOH.

A water soluble or dispersible, solid plasticizer is used to improve the flow characteristics of the melt extrudable composition during the injection molding process and to reduce moisture sensitivity and increase modulus of the injection molded article, especially of a tampon applicator. A water dispersible, solid plasticizer is preferred since it better reduces the moisture sensitivity of the article. By "solid plasticizer" we mean a material that modifies the flow properties of polyvinyl alcohol and is solid at temperatures below about 25°C. The plasticizer should also be compatible with the PVOH and may be a solid polyhydric alcohol, most preferably sorbitol, mannitol, pentaerythritol or dipentaerythritol, or other solid hydrophilic compounds known in the art that modifies the flow properties of and possesses compatibility with the PVOH. The amount of the solid plasticizer added to the composition ranges from 1 to 10 wt%, preferably 3 to 8 wt% and, most preferably, about 5 wt%, based on PVOH.

By itself the solid plasticizer does not reduce the melt viscosity to the extent necessary to make thin wall articles by injection molding. The solid plasticizer is added primarily as a modulus enhancer. Also, the solid plasticizer the Tg of the PVOH composition yielding a brittle final product.

A liquid plasticizer such as a liquid polyhydric alcohol is thus added to the composition as the main plasticizer to obtain the necessary flow characteristics for injection molding the thin wall article (tampon applicator) and to improve the flexibility, i.e., reduce the brittleness of the final article. The liquid polyhydric alcohol may be glycerol, ethylene glycol, polyethylene glycol or other hydrophilic compounds known in the art to possess compatibility with the PVOH. The amount of the liquid plasticizer added to the composition ranges from 8 to 20 wt%, preferably 10 to 15 wt%, and, most preferably, about 12 wt%, based on PVOH.

At greater than 10 wt% solid plasticizer, e.g. pentaerythritol, and low levels of liquid plasticizer and upon aging, the liquid plasticizer exudes onto the surface of the article, especially with fully hydrolyzed PVOH.

Optionally, but desirably, an inorganic filler such as starch, clay or calcium carbonate is added to the composition to further increase the modulus and to reduce the cost of the resin composition. The amount of filler would range from 0 to 50 wt%, preferably 5 to 30 wt%, based on PVOH.

The thermal stability of the PVOH can further be improved through the addition of small amounts of a mineral acid, preferably phosphoric acid, to a high intensity mixer in which PVOH and plasticizer may be blended prior to extrusion.

The PVOH should either be low in residual ash (sodium acetate measured as Na₂O) or treated with the neutralizing mineral acid.

Optionally, but advantageously, a dispersing agent such as glycerol mono-oleate (GMO) is added during the mixing operation. The preferred range of GMO or other dispersing agents added during the high intensity mixing is 0.05 wt% to 1.0 wt%, or more preferred 0.1 to 0.5 wt%.

In addition, the melt extrudable composition can also be blended with suitable pigments or odor maskers and other similar materials commonly used in manufacturing personal care products.

U.S. Patent 5,051,222, which is hereby incorporated by reference, teaches a method for making melt extrudable (thermoplastic) PVOH compositions suitable for use in the present invention. The method comprises providing sufficient energy to a PVOH which is at least partially crystalline to both melt the PVOH and substantially eliminate the crystallinity in the PVOH melt while simultaneously removing energy from the PVOH melt at a rate sufficient to avoid decomposition of the PVOH. The melt is then extruded, preferably into a strand, rapidly cooled and cut into pellets. Such thermoplastic PVOH pellets are marketed by Air Products and Chemicals, Inc. under the trademark VINEX.

In the following description of the process for making a melt extrudable PVOH/solid plasticizer/liquid plasticizer composition according to the invention, it will be understood that any reference to PVOH in preparing melt extrudable PVOH pellets containing liquid plasticizer (according to U.S. 5,051,222) for melt blending with the solid plasticizer would also apply to the direct preparation of the melt extrudable PVOH/solid plasticizer/liquid plasticizer blend.

The PVOH/solid plasticizer compositions are prepared either directly by melt blending a PVOH composition containing liquid plasticizer with a solid plasticizer in a high intensity extruder similarly to U.S. 5,051,222 or indirectly by first preparing melt extruded pellets of the thermoplastic PVOH composition containing a suitable high intensity extruder according to U.S. 5,051,222, and then melt blending with the solid plasticizer to make pellets of the PVOH/solid plasticizer/liquid plasticizer composition. Such pellets may then be converted to the desired injection molded article by an appropriate extruder. Rather than making pellets, the PVOH composition can be melt blended with the solid plasticizer and directly injection molded to the desired article.

The extruder used in the melt compounding of the PVOH, liquid plasticizer and optionally solid plasticizer, must be able to provide an energy input of at least about 0.27 KW hr/kg, preferably 0.3-0.45 KW hr/kg, to the PVOH. The energy required to melt process the solid plasticizer from ambient temperature to the forming temperatures is typically less than 0.1 KW hr/kg solid plasticizer. Since this amount is less than the energy requirements per kg of PVOH, minimal changes in the process conditions for PVOH are necessary. The energy input for melting the PVOH (and solid plasticizer), may be heat or mechanical energy, but with most suitable extruders will be all mechanical energy as will be the sheering energy.

The upper, practical limit of energy input would be about 0.6 KW hr/kg because any energy beyond that necessary to melt the PVOH, and solid plasticizer if present, and to eliminate PVOH crystallinity must be removed as "waste energy". The more energy that passes through the PVOH that has to be removed the more inefficient the process. Approximately 0.1 to 0.15 KW hr/kg is required to melt (and heat) the PVOH and about 0.2 to 0.3 KW hr/kg is needed to sheer the crystalline PVOH areas in the melt.

Further, the extruder must be capable of removing the excess energy input not required in the heating, melting and sheering of the PVOH resin. The excess energy is removed through the extruder barrel, extruder screw or through the evaporation of liquid plasticizer during the devolatilization step. Examples of suitable commercially available extruders include Werner and Pfleiderer twin extruders and kneader-extruders such as the Buss kneaders.

The first step in a preferred method (indirect) for making melt extrudable PVOH/solid plasticizer/liquid plasticizer compositions and the injection molded articles involves the preparation of PVOH blended with a dispersing agent to produce a granular, free flowing mixture to be fed into a melt compounding extruder. The blend is prepared using a variable speed high intensity mixer equipped with the cooling jacket. PVOH is charged to the mixer and the temperature is allowed to rise to approximately 55°C before the GMO is added to the mixing vessel. Next the liquid plasticizer (glycerol) is injected into the mixing chamber under pressure through a spray nozzle once 70°C is reached. The nozzle serves to atomize the plasticizer and eliminate clumping of the PVOH. During the addition of the liquid plasticizer, both the cooling jacket temperature and the mixer speed are adjusted to maintain the temperature of the mix below 105°C, preferably near 95°C. Advantageously, the required amount of mineral acid, preferably phosphoric acid, is mixed with the plasticizer in a liquid blend.

Other solid or liquid additives, pigments, fillers or stabilizers can be added once the plasticizer addition is complete. The mixing action is continued until a free flowing homogeneous product is achieved. This is generally 4-10 minutes but can vary depending upon the addition rate of the glycerol and the Tg of the PVOH polymer. After a free flowing mix is produced, it is discharged into a cooling blender and the temperature reduced to 30°C. The product is ready for extrusion compounding with the solid plasticizer and injection molding into the desired article.

Rather than performing a premixing step, it is more desirable to directly inject a liquid plasticizer (glycerol), mineral acid (H₃PO₄) and dispersing agent (GMO) into the extruder at about the 3 diameter distance downstream from the feed location using the first diameters to heat up the PVOH. Thus, the additives are blended into the PVOH which is then quickly melted, sheered and extruded, avoiding a more prolonged exposure to high heat in the premixer.

The preferred extruder is capable of achieving a high level of mechanical energy input, uniformly distributed through the polymer. The mechanical energy input of the screw extruder can be quantified by measuring the specific energy. The specific energy input of a screw extruder is computed from the ratio of electrical energy, in kilowatts (KW), of the screw mechanical drive to the throughput rate of the polymer (kg/hr). The preferred specific, or mechanical, energy input for the preparation of a homogeneous PVOH melt is greater than about 0.3 KW hr/kg. The extruder must also have cooling capabilities, most preferably jacketing in the barrel sections for heat transfer oil or water. The preferred temperature profile of the PVOH obtained in the different extruder zones is 150-230°C depending upon the grade of PVOH, most preferred 170-220°C. Temperatures less than this result in the appearance of unmelted particles in the strands from the extruder outlet, while temperatures above this range increase the number of gels in the strand and promote degradation of the polymer at the barrel walls.

The thermoplastic PVOH pellets are then melt blended with the solid plasticizer in a conventional single or twin extruder under normal extrusion conditions.

Although the tampon applicator of the present invention can comprise a single elongated tubular member, in preferred embodiments the tampon applicator of the present invention comprises a pair of telescoping tubes, either or both of which can be straight or curved. If both tubes are curved, the radii of curvature can be the same or different, but most preferably the tubes will have a mating relationship with the common radius of curvature. The tubes can be prepared by injection molding, extrusion, or by both injection molding and extrusion. If both injection molding and extrusion are employed, the outer tube typically will be made by injection molding whereas the inner tube will be made by extrusion. Finally, either or both tubes can be made from the melt extrudable PVOH/solid plasticizer/liquid plasticizer composition.

The molding or extrusion of the PVOH/solid plasticizer/liquid plasticizer composition in accordance with the present invention can be performed using conventional equipment in accordance with well-known procedures. The specific processing conditions employed will, of course, depend upon the specifics of the equipment and the molds or dies used. For a general discussion of injection molding, see Irvin I. Rubin, *"Injection Molding",* in Herman F. Mark, et al., Editors, *"Encyclopedia of Polymer Science and Engineering",* Vol 8, John Wiley & Sons, New York, 1987, pp 102-138. For a general discussion of extrusion, see George A. Kruder, *"Extrusion",* in Herman F. Mark, et al., Editors, *"Encyclopedia of Polymer Science and Engineering",* Vol 6, John Wiley & Sons, New York, 1986, pp 571-631.

For purposes of illustration, a typical injection molding apparatus comprises a rear zone into which the polymer is introduced, a central zone, a front zone, a nozzle and the mold. With such an apparatus, typical injection molding process temperatures are as follows:

| Zone | °C |
|---|---|
| Nozzle | 191-193 |
| Front | 191-210 |
| Center | 185-204 |
| Rear | 143-193 |
| Mold | 29-49 |

Other typical processing conditions include an injection pressure on the order of 400 to 900 psi (2800 to 6300 KPa), a holding pressure on the order of 400 to 1600 psi (2800 to 11,200 KPa), and a hold time on the order of 1.5 to 2.0 seconds.

The PVOH/solid plasticizer/liquid plasticizer compositions in the following examples were prepared either directly by melt blending a PVOH composition containing liquid plasticizer and phosphoric acid with the solid plasticizer in a 46 mm reciprocating, rotating Buss kneader or indirectly by first preparing pellets of a melt extrudable PVOH composition containing liquid plasticizer and phosphoric acid in a suitable extruder and then melt blending the thermoplastic PVOH pellets with the solid plasticizer in single or twin screw extruders.

In either case, the pellets of the PVOH/solid plasticizer composition were injection molded into tensile and Izod bars using a Boy 50 ton (4.5 x 10⁴ kg) injection molding machine equipped with a standard ASTM test part mold. These bars were then tested for various physical and mechanical properties.

In the following examples all parts are parts by weight.

### EXAMPLE 1

The following blend was prepared in a 400 liter high intensity mixer by first adding Airvol 205 PVOH. The blender was started and the PVOH was heated to 50°C at which point the GMO was added. Next, phosphoric acid (687g) was added to the glycerine and mixed thoroughly. This mixture was slowly added to the PVOH while the temperature was maintained below 100°C. The resulting PVOH mixture was discharged into a 1200 liter cooling blender when all the liquid had been added and the material was free flowing. Just prior to discharging the mixture into the cooler, the titanium dioxide was added.

| Component | wt(g) |
|---|---|
| Airvol 205 | 113,500 |
| TiO₂ | 3,862 |
| Glycerine | 6,229 |
| GMO | 959 |

This PVOH blend was loaded into a volumetric screw feeder which was calibrated for mass flow as a function of screw feed. Similarly, a second feeder was filled with calcium carbonate and a third feeder was charged with pentaerythritol. Both feeders were similarly calibrated.

A 46 mm (11 L/D) reciprocating screw extruder (Buss-Condux) was set up to have a feed port at 1 L/D, a second feed port at 5 L/D and vacuum venting port at 7 L/D and discharged into a gear pump at 11 L/D. The PVOH mixture and pentaerythritol were fed into the first feed port while the calcium carbonate was fed into the second feed port. The Zenith gear pump was used to pump glycerine into the extruder just after the first feed port. A second pump head was used to pump polyethylene glycol-400 (PEG-400) into the extruder just after the second feed port. The final composition of the resulting blend is shown below:

| Component | wt(%) |
|---|---|
| Airvol 205 | 59.5 |
| Pentaerythritol | 10.0 |
| Glycerine | 6.5 |
| GMO | 0.5 |
| PEG-400 | 6.5 |
| TiO₂ | 2.0 |
| CaCO₃ | 15.0 |

The extruder was operated on the following conditions:

| | |
|---|---|
| Zone 1 (screw) | 225°C |
| Zone 2 (barrel) | 225°C |
| Zone 3 (barrel) | 195°C |
| Zone 4 (gear pump) | 160° C |
| Die | 154° C |
| Screw Speed | 315 RPM |
| Discharge Pressure | 1380 KPa |
| Throughput | 20.4 kg/hr |
| Screw Power | 4.3 KW |
| Vacuum | 705 mm Hg |

The extruder strands were cooled on chilled rolls and chopped into pellets. The pellets were injected molded in a standard spiral flow test mold installed in a 50 ton Boy injection molding machine. The screw barrel temperatures were set at 210° C and the injection pressure varied from 11,000 KPa to 9,310 KPa. The mold temperature was similarly varied from 40 to 60° C. The results are shown below:

| Injection Pressure (kPa) | Mold Temp (° C) | Spiral Flow (cm) |
|---|---|---|
| 9310 | 40 | 9.0 |
| 11000 | 40 | 10.6 |
| 9310 | 60 | 9.2 |
| 11000 | 60 | 11.0 |

### Example 2

In this Example several melt extrudable PVOH compositions containing various amounts of pentaerythritol were prepared following a procedure similar to that of Example 1. These compositions were then molded into tensile bars and conditioned at 50% relative humidity for four weeks prior to testing. The testing was conducted according to ASTM D638 procedure. The results are show in Table 1.

**TABLE 1**

| **RUN** | **A-205 (wt%)** | **GMO (wt%)** | **Glycerin (wt%)** | **Pentaerythritol (wt%)** | **Tensile Break (MPa)** | **Elongation Break %** |
|---|---|---|---|---|---|---|
| 1 | 89.5 | 0.5 | 10 | 0.00 | 32 | 110 |
| 2 | 89.5 | 0.5 | 7.5 | 2.5 | 34 | 90 |
| 3 | 89.5 | 0.5 | 5 | 5 | 37 | 80 |
| 4 | 89.5 | 0.5 | 2.5 | 7.5 | 40 | 70 |
| 5 | 89.5 | 0.5 | 0.00 | 10 | 51 | 60 |

The data in Table 1 indicate that the presence of solid plasticizer increases the tensile strength and reduces the elongation of the parts as desired for a tampon applicator.

### STATEMENT OF INDUSTRIAL APPLICATION

The present invention provides thermoformed, especially injection molded, articles of a melt extrudable PVOH/solid plasticizer composition and, in particular, injection molded thin walled articles such as tampon applicators.

## Claims

1. In a thermoformable polyvinyl alcohol composition containing plasticizer and optionally filler, the improvement which comprises 1-10 wt% solid plasticizer for the polyvinyl alcohol and 8-20 wt% liquid plasticizer for the polyvinyl alcohol, the amount of plasticizers based on polyvinyl alcohol.

2. The composition of Claim 1 which contains 3-8 wt% solid plasticizer.

3. The composition of Claim 1 which contains about 5 wt% solid plasticizer.

4. The composition of Claim 1 in which the solid plasticizer is selected from the group consisting of sorbitol, mannitol, pentaerythritol, dipentaerythritol and mixtures thereof.

5. A thermoformable composition which consists essentially of
(a) a thermoplastic polyvinyl alcohol, the polyvinyl alcohol made melt extrudable by adding to the polyvinyl alcohol at least 0.27 KW hr/kg of polyvinyl alcohol to both melt it and substantially eliminate the crystallinity in the melt while simultaneously removing energy from the melt at a rate sufficient avoid decomposition of the polyvinyl alcohol,
(b) 1-10 wt% solid plasticizer for the polyvinyl alcohol, the solid plasticizer being selected from the group consisting of sorbitol, mannitol, pentaerythritol, dipentaerythritol and mixtures thereof,
(c) 8-20 wt% liquid plasticizer for the polyvinyl alcohol, the liquid plasticizer being selected from the group consisting of glycerol, ethylene glycol, polyethylene glycol and mixtures thereof, and
(d) 0-50 wt% filler, the amount of plasticizers and filler being based on polyvinyl alcohol.

6. The composition of Claim 5 which contains 3-8 wt% solid plasticizer.

7. The composition of Claim 5 which contains about 5 wt% solid plasticizer.

8. The composition of Claim 5 in which the liquid plasticizer is selected from the group consisting of glycerol, ethylene glycol, polyethylene glycol and mixtures thereof.

9. The composition of Claim 5 in which the thermoformable composition contains 5-30 wt% filler which is starch, clay or calcium carbonate.

10. A thin wall thermoprocessed article prepared from the thermoformable composition of Claim 5.

11. A thin wall thermoprocessed article prepared from the thermoformable composition of Claim 6.

12. A thin wall thermoprocessed article prepared from the thermoformable composition of Claim 7.

13. A thin wall thermoprocessed article prepared from the thermoformable composition of Claim 8.

14. A thin wall thermoprocessed article prepared from the thermoformable composition of Claim 9.

15. The article of Claim 10 which is a tampon applicator comprising at least one elongated tubular member.

16. The article of Claim 15 in which the applicator comprises a pair of telescoping tubes, at least one of which is prepared from the thermoformable composition.

17. In a water-dispersible insertion device for tampons and the like comprising at least one thin walled tubular member in which the walls are comprised of a thermoformed, water soluble polyvinyl alcohol compounded with a plasticizer, the improvement which comprises the polyvinyl alcohol made melt extrudable by adding to the polyvinyl alcohol 0.3 to 0.45 KW hr/kg to both melt it and substantially eliminate the crystallinity in the melt while simultaneously removing energy from the melt at a rate sufficient avoid decomposition of the polyvinyl alcohol, 1-10 wt% solid plasticizer being selected from the group consisting of sorbitol, mannitol, pentaerythritol, dipentaerythritol and mixtures thereof and 8-20 wt% liquid plasticizer.

18. The insertion device of Claim 17 which further comprises 0-50 wt% starch, clay or calcium carbonate filler.

19. The insertion device of Claim 18 in which the plasticizer is pentaerythritol.

20. The insertion device of Claim 19 in which the filler is calcium carbonate.

21. In an article of manufacture comprising a thermoformed, water soluble polyvinyl alcohol compounded with a plasticizer, the improvement which comprises polyvinyl alcohol that has been made melt extrudable by adding to the polyvinyl alcohol at least 0.27 KW hr/kg to both melt it and substantially eliminate the crystallinity in the melt while simultaneously removing energy from the melt at a rate sufficient to avoid decomposition of the polyvinyl alcohol and the plasticizer being selected from the group consisting of sorbitol, mannitol, pentaerythritol, dipentaerythritol and mixtures thereof.
